# EUROPÄISCHE PATENTSCHRIFT

(11) **EP 1 196 604 B1**
(45) Veröffentlichungstag und Bekanntmachung des Hinweises auf die Patenterteilung: **27.10.2004**
(21) Anmeldenummer: 00956173.9
(22) Anmeldetag: 10.07.2000
(51) Int. Cl.: C12N 15/53, C12N 9/02, C12P 7/64, C07C 69/732, C07C 59/42, C07C 409/04

(54) **11-ARACHIDONAT-LIPOXYGENASE-MUTANTE**
11-ARACHIDONATE-LIPOXYGENASE MUTANTS
MUTANTS DE 11-ARACHIDONATE-LIPOXYGENASE

(30) Priorität: 08.07.1999 DE 19931819
(43) Veröffentlichungstag der Anmeldung: 17.04.2002
(73) Patentinhaber: Institut für Pflanzenbiochemie-IPB, 06120 Halle (Saale) (DE)
(72) Erfinder: FEUSSNER, Ivo, 06114 Halle (DE); HORNUNG, Ellen, 06484 Quedlinburg (DE); ROSAHL, Sabine, 06114 Halle (DE)
(74) Vertreter: Grünecker, Kinkeldey, Stockmair & Schwanhäusser Anwaltssozietät
(86) Internationale Anmeldenummer: PCT/EP2000/006539
(87) Internationale Veröffentlichungsnummer: WO 2001/004323

(56) Entgegenhaltungen:
- WO-A-00/60093
- MULLIEZ E ET AL: "5 LIPOXYGENASE FROM POTATO TUBERS IMPROVED PURIFICATION AND PHYSICOCHEMICAL CHARACTERISTICS" BIOCHIMICA ET BIOPHYSICA ACTA, Bd. 916, Nr. 1, 1987, Seiten 13-23, XP000990244 ISSN: 0006-3002
- REDDY G RAMAKRISHNA ET AL: "11-Hydroperoxyeicosatetraenoic acid is the major dioxygenation production of lipoxygenase isolated from hairy root cultures of Solanum tuberosum." BIOCHEMICAL AND BIOPHYSICAL RESEARCH COMMUNICATIONS, Bd. 189, Nr. 3, 1992, Seiten 1349-1352, XP002162902 ISSN: 0006-291X
- REDDY C C ET AL: "MECHANISM OF FORMATION OF LEUKOTRIENES AND LIPOXINS FROM ARACHIDONIC ACID CATALYZED BY HOMOGENOUS LIPOXYGENASE FROM POTATO TUBERS" ADVANCES IN PROSTAGLANDIN THROMBOXANE AND LEUKOTRIENE RESEARCH, 1989, Seiten 132-136, XP000990260 ISSN: 0732-8141
- VAN ZADELHOFF GUUS ET AL: "With anandamide as substrate plant 5-lipoxygenases behave like 11-lipoxygenases." BIOCHEMICAL AND BIOPHYSICAL RESEARCH COMMUNICATIONS, Bd. 248, Nr. 1, 9. Juli 1998 (1998-07-09), Seiten 33-38, XP002162903 ISSN: 0006-291X
- DI MARZO VINCENZO ET AL: "Biosynthesis, structure and biological activity of hydroxyeicosatetraenoic acids in Hydra vulgaris." BIOCHEMICAL JOURNAL, Bd. 295, Nr. 1, 1993, Seiten 23-29, XP000990275 ISSN: 0264-6021
- DI MARZO VINCENZO ET AL: "Polyunsaturated-fatty-acid oxidation in Hydra: Regioselectivity, substrate-dependent enantioselectivity and possible biological role." BIOCHEMICAL JOURNAL, Bd. 300, Nr. 2, 1994, Seiten 501-507, XP000990271 ISSN: 0264-6021
- LEITZ THOMAS ET AL: "Enantiospecific synthesis of bioactive hydroxyeicosatetraenoic acids (HETEs) in Hydra magnipapillata." BIOCHIMICA ET BIOPHYSICA ACTA, Bd. 1213, Nr. 2, 1994, Seiten 215-223, XP000990267 ISSN: 0006-3002
- HAWKINS D J ET AL: "RESOLUTION OF ENANTIOMERS OF HYDROXYEICOSATETRAENOATE DERIVATIVES BY CHIRAL PHASE HIGH-PRESSURE LIQUID CHROMATOGRAPHY" ANALYTICAL BIOCHEMISTRY, Bd. 173, Nr. 2, 1988, Seiten 456-462, XP000990251 ISSN: 0003-2697
- KUEHN H ET AL: "ANALYSIS OF THE STEREOCHEMISTRY OF LIPOXYGENASE-DERIVED HYDROXYPOLYENOIC FATTY ACIDS BY MEANS OF CHIRAL PHASE HIGH-PRESSURE LIQUID CHROMATOGRAPHY" ANALYTICAL BIOCHEMISTRY, Bd. 160, Nr. 1, 1987, Seiten 24-34, XP000990252 ISSN: 0003-2697
- PORTER N A ET AL: "THE RESOLUTION OF RACEMIC HYDROPEROXIDES A CHROMATOGRAPHY-BASED SEPARATION OF PERKETALS DERIVED FROM ARACHIDONIC LINOLEIC AND OLEIC ACID HYDROPEROXIDES" CHEMICAL RESEARCH IN TOXICOLOGY, Bd. 3, Nr. 3, 1990, Seiten 236-243, XP000990214 ISSN: 0893-228X
- HORNUNG E ET AL: "CONVERSION OF CUCUMBER LINOLEATE 13-LIPOXYGENASE TO A 9-LIPOXYGENATING SPECIES BY SITE-DIRECTED MUTAGENESIS" PROCEEDINGS OF THE NATIONAL ACADEMY OF SCIENCES OF USA,US,NATIONAL ACADEMY OF SCIENCE. WASHINGTON, Bd. 96, Nr. 7, 1999, Seiten 4192-4197, XP000915201 ISSN: 0027-8424
- FEUSSNER IVO ET AL: "Lipoxygenase catalyzed oxygenation of lipids." FETT, Bd. 100, Nr. 4-5, Mai 1998 (1998-05), Seiten 146-152, XP002162906 ISSN: 0931-5985

## Beschreibung

Die vorliegende Erfindung betrifft ein Verfahren zur Herstellung einer pflanzlichen Lipoxygenase mit veränderter Positionsspezifität sowie die durch das Verfahren erhaltene Lipoxygenase und deren Verwendung zur Hydroperoxylierung von Arachidonsäure an Kohlenstoffatom 11.

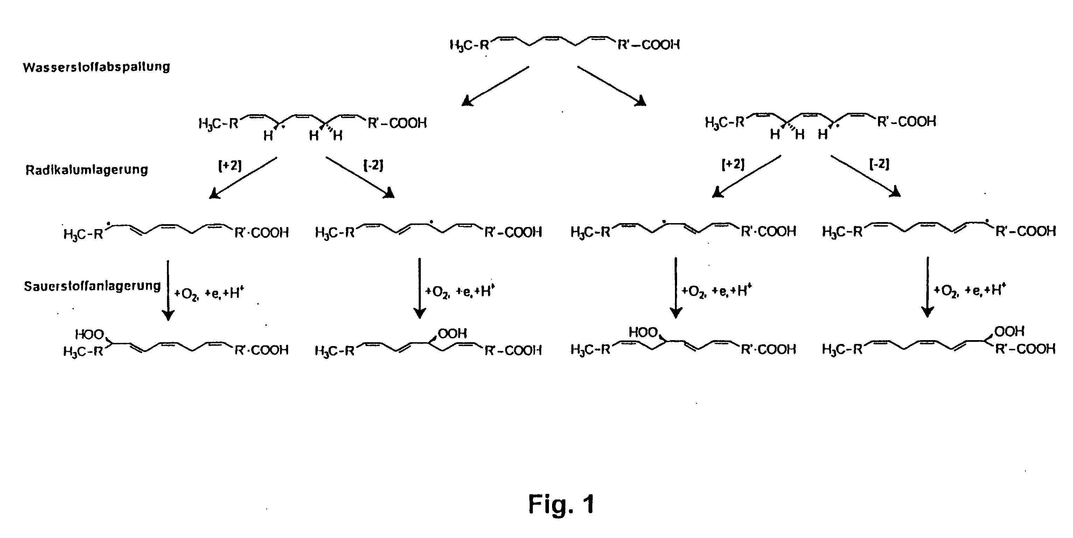

Lipoxygenasen (LOXs, Linolensäure: Sauerstoff-Oxidoreduktase; EC.1.13.11.12; LOXs) sind im Pflanzen- und Tierreich weit verbreitet (Siedow, J.N. (1991) Ann. Rev. Plant Physiol. Plant Mol. Biol. 42, 145-188; Yamamoto, S. (1992) Biochim. Biophys. Acta 1128, 117-131). Diese Enzyme stellen eine Familie aus eisenhaltigen Dioxygenasen dar, die eine bereichs- (oder positions-) und stereoselektive Oxygenierung von Polyenfettsäuren zu Hydroperoxyderivaten katalysieren (Rosahl, S. (1996) Z. Naturforsch. 51c, 123-138). In Säugern werden LOXs nach ihrer Spezifität für bestimmte Positionen bei der Arachidonsäureoxygenierung klassifiziert (Yarnamoto, S. (1992) Biochim. Biophys. Acta 1128, 117-131; Schewe, T., Rapaport, S.M. & Kühn, H. (1986) Adv. Enzymol. Mol. Biol 58, 191-272). So wurden hier bisher 15-, 12-, 8- und 5-LOXs isoliert und charakterisiert. LOXs, die die Insertion von Sauerstoff an den Positionen 9- bzw. 11 am Kohlenstoffgerüst von Arachidonsäure inserieren, sind noch nicht bekannt (Yamamoto, S. (1992) Biochim. Biophys. Acta 1128, 117-131). Da Arachidonsäure in höheren Pflanzen nicht vorkommt oder nur in geringen Mengen als Bestandteil von Speicherlipiden, werden LOXs aus Pflanzen als 9- und 13-LOXs klassifiziert. Diese Nomenklatur leitet sich von der Position ab, an der in Linolsäure (LA) die Oxygenierung erfolgt (Gardner, H.W. (1991) Biochim. Biophys. Acta 1084, 221-239). In jüngster Zeit ist eine umfangreichere Klassifizierung pflanzlicher LOXs auf der Grundlage eines Vergleichs der Primärstrukturen vorgeschlagen worden (Shibata, D. & Axelrod, B. (1995) J. Lipid Mediators Cell Signal. 12, 213-228). Die Spezifität einer LOX für eine bestimmte Position ist das Ergebnis zweier katalytischer Teilreaktionen:
(i)
   der bereichs- und stereospezifischen Entfernung von Wasserstoff, wobei bei Fettsäuren, die mehrere Doppelbindungen enthalten (wie Linolensäure, Arachidonsäure oder Eikosapentaensäure), die Wasserstoffentfemung an verschiedenen Positionen erfolgen kann;
(ii)
   der bereichs- und sterospezifischen Sauerstoff-Insertion (wobei der Sauerstoff an verschiedenen Positionen (der +2 oder -2 Position) eingefügt werden kann (Vergleich Figur 1). Somit kann eine Fettsäure mit 3 doppelallylischen Methylenen, wie Arachidonsäure, von einer LOX zu 6 regioisomeren Hydroperoxyderivaten (HPETEs) oxygeniert werden, nämlich zu 15- und 11-HPETE (diese stammen aus der Entfernung von Wasserstoff an Position C-13), 12- und 8-HPETE (diese stammen aus der Wasserstoffentfernung an Position C-10) und 9- und 5-HPETE (diese stammen aus der Wasserstoffentfemung an Position C-7). Experimente mit 12- und 15-LOX aus Säugern zeigten, daß die Position der Wasserstoffentfemung verändert werden kann, wenn kritische Aminosäuren durch gerichtete Mutagenese verändert werden (Borngräber, S., Kuban, R. J., Anton, M. & Kühn, H. (1996) J. Mol. Biol. 264, 1145-1153; Sloane, D.L., Leung, R., Craik, C. S. & Sigal, E (1991) Nature 354, 149-152). Versuche zum Ändern der LOX-Reaktivität von einer +2 nach -2-Umlagerung oder umgekehrt (z. B. Umwandeln einer Linoleat-13-LOX zu einer 9-LOXs) mit Hilfe gerichteter Mutagenese waren kürzlich erfolgreich (Hornung, E., Walther, M., Kühn, H. & Feussner, I. (1999) Proc. Natl. Acad. Sci. USA 96, 4192-4197).

Der vorliegenden Erfindung lag das technische Problem zugrunde, ein Verfahren anzugeben, mit der eine LOX mit gewünschter C-11 Positionsspezifität bei Arachidonsäure bereitgestellt werden könnte.

Dieses Problem wird erfindungsgemäß gelöst durch ein Verfahren, bei dem mindestens eine Aminosäure in einer Wildtyp-LOX, vorzugsweise aus der Kartoffelknolle ausgetauscht wird.

Figur 1 zeigt die Spezifität einer LOX-Reaktion mit Substraten, die zwei allylische Methylene enthalten.

Figur 2 zeigt die HPLC-Analyse von Hydroxyfettsäuren, die mit Hilfe der Wildtyp-LOX aus Kartoffelknollen und der V576F-Mutante aus Arachidonsäure nach Reduktion der Hydroperoxyfettsäuren mit Natriumborhydrid erhalten werden.

Figur 3 zeigt die Sequenz der Wildtyp-LOX aus Kartoffelknollen. Die mutageniserte Aminosäureposition ist unterstrichen. Die verwendeten Primer 1 und 2 sind ebenfalls gezeigt.

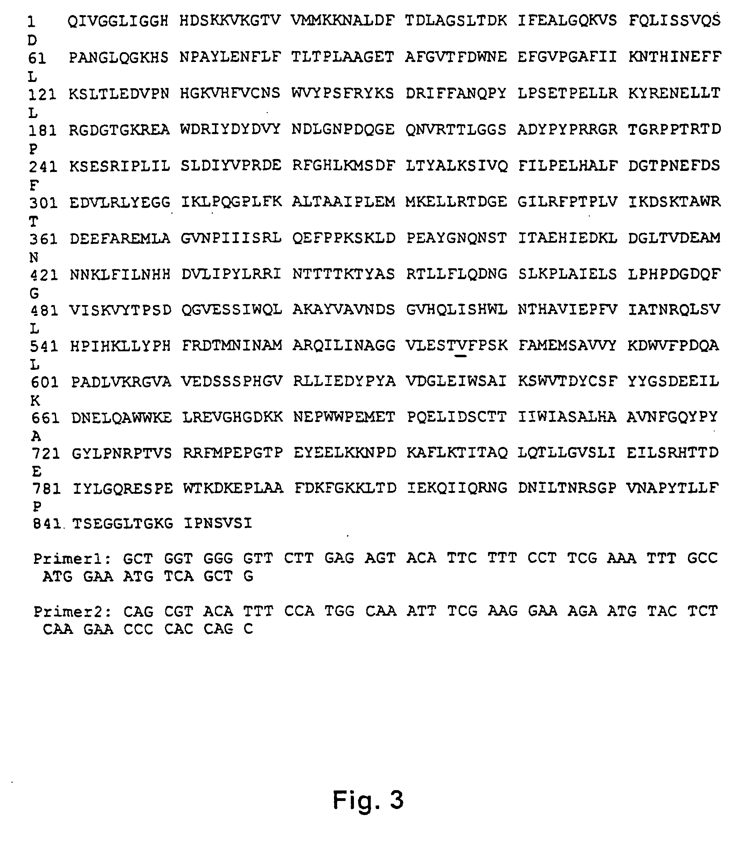

Bei einer bevorzugten Ausführungsform erfolgt der Austausch der Aminosäuren im Bereich der Aminosäureposition 570 bis 581 der LOX aus Kartoffelknollen. Die oben angegebenen Aminosäurepositionen beziehen sich auf die Sequenz unter der Zugangsnummer S73865 in der EMBL-Datenbank bzw. der Sequenz gemäß Figur 3. Die zu den Aminosäurepositionen 593 bis 602 der Lipoxygenase aus *Cucumis sativus* korrespondierenden Positionen in LOXs aus anderen Pflanzenarten können durch Sequenzvergleiche zwischen der Sequenz X92890 und den weiteren Proteinsequenzen wie aus Sojabohnen, Kartoffel, Arabidopsis, Tabak oder Gerste leicht ermittelt werden. Die folgende Tabelle 1 zeigt das Ergebnis eines Aminosäurevergleichs zwischen dem aus Gurken stammenden Enzym und den korrespondierenden Positionen in den Enzymen aus anderen Pflanzen. Die erste Gruppe (15-LOX) zeigt einen Vergleich zwischen LOXs, die an Position 15 eine Hydroperoxy-Gruppe in ein Molekül Arachidonsäure einführen, während die zweite Gruppe (5-LOX) einen Vergleich zwischen Sequenzen zeigt, die an Position 5 einen Hydroperoxy-Rest einführen.

**Tabelle 1**

| **Vergleich der Aminosäurereste, die an der Spezifität einer pflanzlichen LOX für eine bestimmte Position (15 bzw. 5) beteiligt sind.** | | | |
|---|---|---|---|
| **ENZYME** **Rest** | **Zugangs-Nr.** | **Position d. AS-Restes** | **AS** |
| **15-LOX** | | | |
| Gurke-Lipid-Körper LOX | X92890 | 596/597 | Thr/His |
| LOX-1 aus Sojabohnensamen | P08170 | 556/557 | Thr/Phe |
| LOX-H1 aus Kartoffeln | X96405 | 614/615 | Ser/Phe |
| LOX-2 aus *Arabidopsis* | P38418 | 611/612 | Cys/Phe |

| **5-LOX** | | | |
|---|---|---|---|
| LOX aus Kartoffel | S73865 | 575/576 | Thr/Val |
| Elicitor-induzierte LOX aus *Tabak* | X84040 | 580/581 | Thr/Val |
| LOX-A aus Gerstenkorn | L35931 | 574/575 | Thr/Val |

Das Sequenzmotiv bei Position 570 bis 581 lautet GVLESTVFPSK (Sequenz gemäß S73865).

Bei einer besonders bevorzugten Ausführungsform erfolgt der Austausch an Position 576 der Sequenz S73865. An Position 576 befindet sich im Wildtyp ein Val-Rest. Hier wird der Rest an Position 576 durch einen Phe-Rest ersetzt. Dabei führt der Austausch in dem Bereich der Aminosäureposition 570 bis 581 dazu, daß die 5-LOX aus der Kartoffelknolle in eine Arachidonsäure 11-LOX umgewandelt wird. Im folgenden wird diese Mutante auch als V576F bezeichnet. Die Wildtypsequenz ist als Figur 3 gezeigt. Die Position 576 ist markiert.

Vorzugsweise erfolgt der Austausch der Aminosäuren in dem Wildtyp mit Hilfe der gerichteten Mutagenese, wie sie im Stand der Technik hinlänglich bekannt ist.

Die vorliegende Erfindung betrifft weiterhin LOX-Mutanten, die nach den oben beschriebenen Verfahren erhältlich sind. Die erfindungsgemäßen LOXs lassen sich mit Hilfe der aus dem Stand der Technik bekannten Verfahren, wie der gerichteten Mutagenese, und der anschließenden Proteinexpression herstellen. Dabei gelten als erfindungsgemäß insbesondere die Mutanten, die nach Inkubation mit Arachidonsäure zu mindestens 40 %, vorzugsweise 50 % das an Position 11 perhydroxylierte Derivat liefern.

Die vorliegende Erfindung betrifft femer Nukleinsäuren, die für die erfindungsgemäßen LOXs kodieren. Ausgehend von den im Stand der Technik verfügbaren Wildtypsequenzen, lassen sich die erfindungsgemäßen Sequenzen durch gerichtete Mutagenese herstellen.

Femer betrifft die vorliegende Erfindung Vektoren, in die die erfindungsgemäßen Nukleinsäuren zum Zwecke der Klonierung und Expression eingebracht werden. Entsprechende Klonierungs- und Expressionsvektoren sind dem Fachmann aus dem Stand der Technik hinlänglich bekannt (vgl. Maniatis et al. Molecular Cloning, A Laboratory Manual (1989), Cold Spring Hator Laboratory Press).

Die vorliegende Erfindung betrifft femer eine Zelle, in die die erfindungsgemäße Nukleinsäure oder der erfindungsgemäße Vektor eingebracht werden. Nach Einbringen der Nukleinsäure bzw. des Vektors ist die Zelle dann in der Lage, eine LOX erstmalig oder in verstärktem Maße zu exprimieren. Auf diese Weise kann das Fettsäuremuster einer Zelle gezielt verändert werden mit dem Ergebnis, daß der Phänotyp der Zelle in verschiedener Hinsicht verändert werden kann. Hierzu zählt u. a. eine andere Zusammensetzung der Zellmembran.

Schließlich können durch *in vitro* -Kultivierungsverfahren aus den o. g. Zellen neue Pflanzen bzw. Pflanzenteile regeneriert werden. Zum Herstellen solcher transgener Pflanzen kann beispielsweise das bekannte Transformationssystem auf der Basis von *Agrobakterien* und Ti-Plasmid-Derivaten eingesetzt werden.

Die erfindungsgemäßen LOXs erlauben erstmalig das Herstellen neuer Arachidonsäure-Derivate in großem Maßstab. Hierzu wird Arachidonsäure als Substrat mit den erfindungsgemäßen LOX unter geeigneten Bedingungen inkubiert. Es erfolgt dann eine Hydroperoxylierung der Arachidonsäure vorzugsweise an Position 11.

Besonders bevorzugt ist ein Arachidonsäure-Derivat, das eine Hydroperoxygruppe an Position 11 enthält. Das Derivat kann dann einfach in das Hydroxyderivat überführt werden. Das so zugängliche 11 S-HPETE kann zur Herstellung der unten gezeigten Alkohole, Aldehyde und Dikarbonsäuren verwendet werden. Das Enzym Hydroperoxid-Lyase ist z.B. in Extrakten von Gurkenkeimlingen enthalten. 2E- und 3Z-Nonenal und deren Alkohole sind wichtige Aromastoffe von Lebensmitteln (z.B. Gurken).

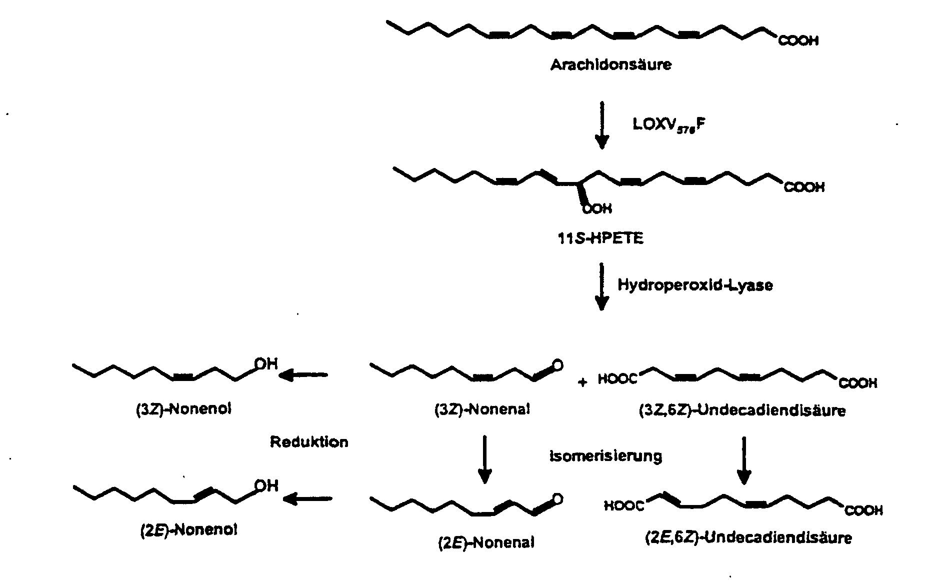

Ein solches Arachidonsäurederivat war bisher nicht zugänglich, da es an einer LOX mit geeigneter Positionsspezifität fehlte.

Die weiteren Beispiele dienen der Erläuterung der Erfindung.

### 1. Herstellen der Mutante V576F

### Materialien:

Die verwendeten Chemikalien wurden aus den folgenden Quellen bezogen: die Standards für chirale und racemische Hydroxyfettsäuren wurden von Chayman Chem (Ann Arbor, Mi, USA) bezogen. Methanol, Hexan, 2-Propanol (allesamt HPLC-Grad) wurden von Baker (Griesheim, Deutschland) bezogen. Restriktionsenzyme wurden von New England BioLabs (Schwalbach, Deutschland) bezogen.

### Gerichtete Mutagenese und Proteinexpression:

Für die bakterielle Expression der Wildtyp-LOX und der LOX-Mutante und für die gerichtete Mutagenese wurde das Plasmid pet3b (Novagen, Deutschland) verwendet, das die cDNA der LOX aus Kartoffelknollen als Insert enthielt (pET-LOX1; vgl. Geerts, A, Feltkamp, D, Rosahl, S (1994) Expression of lipoxygenase in wounded tubers of *solanum tuberosum* L. Plant Physiol. 105: 269-277). Die Mutagenese wurde mit Hilfe des QuikChange-Mutagenese-Kits von Stratagene (Heidelberg, Deutschland), durchgeführt. Oligonukleotide mit den geeigneten Basenaustauschen wurden von MWG-Biotech (Ebersberg, Deutschland) bezogen. Zur Analyse der Mutation wurde ein weiterer konservativer Basenaustausch eingeführt, um eine neue Restriktionsspaltstelle zu erzeugen. Weiterhin wurde die Mutation sequenziert, und mindestens fünf verschiedene Bakterienklone wurden exprimiert und für die Untersuchung der enzymatischen Eigenschaften eingesetzt. Die Expression von pET-LOX1-und seiner Mutante wurde gemäß Feussner, I., Bachmann, A., Höhne, M. & Kindl, H. (1998) FEBS Lett. 431, 433-436, durchgeführt. Zellen aus 1-Liter-Kulturen wurden in 5 bis 7 ml Lysispuffer resuspendiert und mit Hilfe einer Ultraschallspitze mit Pulsen für jeweils 30 Sekunden aufgebrochen, und die Zelltrümmer wurden pelletiert.

### Aktivitätsassay und Probenaufbereitung:

Für die Produktanalyse wurden 0,9 ml der Zell-Lysate mit 0,9 mM Arachidonsäure (Endkonzentration) in 100 mM Tris-Puffer pH 7,5 für 30 Minuten bei Raumtemperatur inkubiert. Die Reaktion wurde abgestoppt durch den Zusatz von Natriumborhydrid, um die gebildeten Hydroperoxyfettsäuren zu den entsprechenden Hydroxyverbindungen umzuwandeln. Die Proben wurden auf pH 3 angesäuert, und die Lipide wurden extrahiert (vgl. Bligh, E.G. & Dyer, W. J. (1959) Can. J. Biochem. Physiol. 37, 911-917). Die untere Chloroformphase wurde wiedergewonnen und das Lösungsmittel abgedampft. Das verbleibende Lipid wurde mit 0,1 ml Methanol gelöst und Aliquots wurden der HPLC-Analyse unterzogen.

### Analyse:

Die HPLC-Analyse wurde mit einem Hewlett Packard 1100 HPLC System, gekoppelt an einen Diodendetektor, durchgeführt. Die RP-HPLC der freien Fettsäurederivate wurde auf einer Nucleosil C-18 Säule (Macherey-Nagel, 250 x 4 mm, 5µm Partikelgröße) mit einem Lösungsmittelsystem aus Methanol/Wasser/Essigsäure (85/15/0.1; v/v/v) und einer Flußrate von 1 ml/min durchgeführt. Die Absorption bei 234 nm (Absorption des konjugierten Diensystems der Hydroxyfettsäuren) und bei 210 nm (Polyenfettsäuren) wurden entsprechend aufgezeichnet. Die Direktphasen-HPLC (SP-HPLC) von Hydroxyfettsäurenisomeren wurde auf einer Zorbax SIL Säule (HP, Waldbronn, Deutschland; 250 x 4,6 mm, 5 µm Partikelgröße) mit einem Lösungsmittelsystem aus n-Hexan/2-Propanol/Essigsäure (100/2/0,1, v/v/v) in einer Flußrate von 1ml/min durchgeführt. Die Enantiomer-Zusammensetzung der Hydroxyfettsäuren wurde analysiert mit Hilfe von Chiral-Phasen-HPLC auf einer Chiralcel-OD-Säule (Daicel Chem. Industrie, vertrieben von Baker Chem., Deventer, Niederlande; 250 x 4,6 mm, 5µm Partikelgröße) mit einem Lösungsmittelsystem aus Hexan/2-Propanol/Essigsäure (100/5/0, 1, v/v/v) in einer Flußrate von 1 ml/min. (vgl. Feussner, I., Balkenhohl, T.J., Porzel, A., Kühn, H.& Wasternack, C. (1997) J. Biol. Chem. 272, 21635-21641 ).

### 2. Herstellen der LOX-V576F-Mutante:

Die für die Herstellung dieser Mutante verwendeten Reagenzien und Verfahren waren im wesentlichen wie bereits oben beschrieben. Im folgenden werden einige Abwandlungen der o. g. Verfahren, die speziell an die Herstellung der V576F Mutante angepaßt waren, erläutert.

### Gerichtete Mutagenese und Proteinexpression:

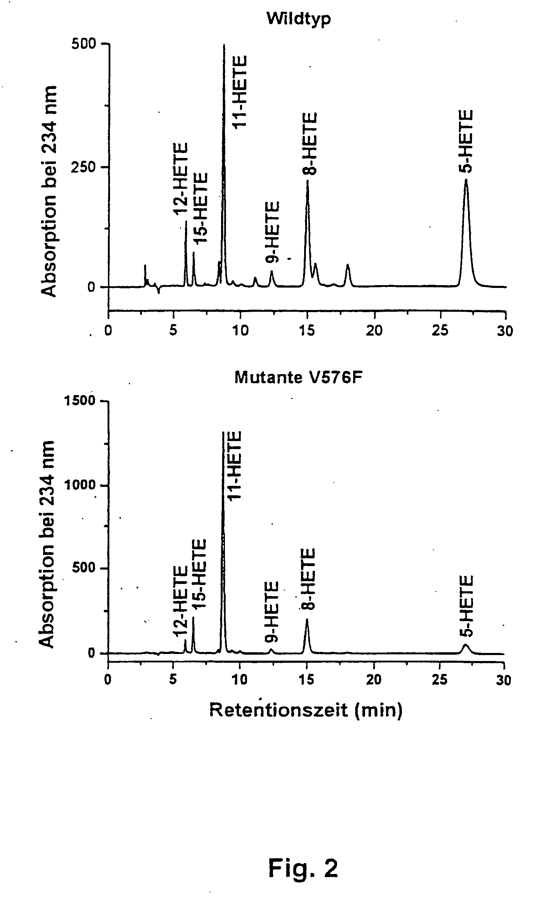

Die Ausgangs-cDNA und der Mutagenesekit waren wie oben beschrieben. Zur Analyse der Mutation wurden weitere konservative Basenaustausche durchgeführt, um eine neue Restriktionsspaltstelle für BstBI zu erzeugen. Für die Herstellung der Mutation V576F wurden die folgenden Primer verwendet: GCT GGT GGG GTT CTT GAG AGT ACA TTC TTT CCT TCG AAA TTT GCC ATG GAA ATG TCA GCT G (kodierender Strang) und CAG CGT ACA TTT CCA TGG CAA ATT TCG AAG GAA AGA ATG TAC TCT CAA GAA CCC CAC CAG C (komplementärer Strang). Weiterhin wurde die Mutante sequenziert und 5 verschiedene Bakterienkolonien wurden expremiert und für die enzymatischen Untersuchungen verwendet. Die Expression von pET-LOX1wurde wie zuvor beschrieben durchgeführt. Auch die weitere Aufbereitung erfolgte wie bereits oben angegeben. Auch die Analyse des erzeugten Fettsäurederivats (das eine Hydroperoxygruppe in 11 Position enthält), erfolgte wie oben angegeben. Das Ergebnis der SP-HPLC Analyse der Umsetzung von Arachidonsäure mit V576F ist in Figur 2 gezeigt. Die folgende Tabelle 2 zeigt einen Vergleich der Spezifität des Wildtyps (wtLOX) mit der Mutante (LOXV₅₇₆F).

**Tabelle 2**

| **Vergleich der Produktspezifität von wtLOX und LOXV**_{**576**}**F mit Arachidonsäure** | | | | | | |
|---|---|---|---|---|---|---|
| **Enzym** | **(15S,13*E*,11*Z*,8*Z*,5*Z*)-20:4** | **(12S, 14*Z*, 10*E*,8*Z*,5*Z*)-20:4** | **(11S,14*Z*,12*E*,8*Z*,5*Z*)-20:4** | **(9*S*,14*Z*,11*Z*,7*E*,5*Z*)-20:4** | **(8*S*,14*Z*,11*Z*9*E*,5*Z*)-20:4** | **(5*S*,14*Z*, 11*Z*,8*Z*6*E*)-20:4** |
| wtLOX | 5 % | 6 % | 23 % | 3 % | 21 % | 42% |
| LOXV₅₇₆F | 9 % | 4 % | 50 % | 3 % | 23 % | 11 % |

### 3. Figurenbeschreibung

**Figur 1** zeigt, daß die Posilionsspezifität der LOX-Reaktion von dem Ort der Wasserstoffabspaltung und von der Orientierung des Radikals abhängt. Die [+2]-Radikalanordnung zeigt, daß der Sauerstoff an dem zweiten Kohlenstoffatom in Richtung des Methylterminus des Substrats, gezählt von der Stelle der Wasserstoffentfernung, eingeführt wird. [-2] zeigt die inverse Orientierung der Radikalanordnung.
**Figur 2** zeigt die HPLC-Analyse von Fettsäuren mit der Mutante V576F. Gleiche Mengen an LOX-Protein wurden mit 0,9 mM Arachidonsäure bei Raumtemperatur für 30 Minuten inkubiert. Nach Reduktion der Lipide mit Natriumborhydrid wurde die Reaktionsmischung auf pH 3 mit Essigsäure angesäuert, und die Upide wurden extrahiert. Die oxygenierten Fettsäurederivate wurden mittels RP-HPLC isoliert, und die einzelnen Positionsisomere wurden mit Hilfe der SP-HPLC analysiert. Die Verhältnisse von S und R wurden mit Hilfe der CP-HPLC analysiert (eingesetzte Abbildungen).
**Figur 3** zeigt die Aminosäuresequenz der Wildtyp-Lipoxygenase aus Kartoffelknollen. Das mutierte Val576 ist unterstrichen.

### Verwendete Abkürzungen sind:

- CP-HPLC: für chirale Phase HPLC;
- RP-HPLC: für Umkehrphasen HPLC;
- SP-HPLC: für Direktphasen HPLC;
- HPETE: für Hydroperoxyarachidonsäure;
- LOX: für Lipoxygenase;
- HETE: für Hydroxyarachidonsäure

## Patentansprüche

1. Verfahren zum Erhöhen der Spezifität einer pflanzlichen Lipoxygenase für Position 11 von Arachidonsäure, umfassend den Schritt:
Austauschen mindestens einer Aminosäure in einer Wildtyp-Lipoxygenase, **dadurch gekennzeichnet, dass** der Austausch an Position 576 der Lipoxygenase aus Kartoffelknollen oder einer korrespondierenden Position in einer Lipoxygenase aus einer anderen Pflanzenart erfolgt, **dadurch gekennzeichnet, dass** der Austausch an Position 576 zum Vorliegen eines Phe-Restes in der Mutante führt.

2. Verfahren nach Anspruch 2, **dadurch gekennzeichnet, dass** der Aminosäureaustausch durch gerichtete Mutagenese herbeigeführt wird.

3. Lipoxygenase, erhältlich durch ein Verfahren nach einem der Ansprüche 1-2.

4. Nukleinsäure, die für eine Lipoxygenase nach Anspruch 3 kodiert.

5. Vektor, enthaltend eine Nukleinsäure nach Anspruch 4.

6. Zelle, enthaltend eine Nukleinsäure nach Anspruch 4 und/oder einen Vektor nach Anspruch 5.

7. Pflanze oder Pflanzenteil, umfassend eine Wirtszelle nach Anspruch 6.

8. Verfahren zum Herstellen von 11-Perhydroxy-Arachidonsäure bzw. dem reduzierten 11-Hydroxyderivat, umfassend den Schritt:
- Umsetzen von Arachidonsäure mit einer Lipoxygenase nach Anspruch 5 und gegebenenfalls Reduzieren der erhaltenen Perhydroxyverbindung zur Hydroxyverbindung.

9. Verwendung einer Lipoxygenase nach Anspruch 3 zum Herstellen von 11-Perhydroxy-Arachidonsäure und/oder 11-Hydroxy-Arachidonsäure.

## Claims

1. A method of enhancing the specificity of a plant lipoxygenase for position 11 of arachidonic acid, comprising the step of
exchanging at least one amino acid in a wild type lipoxygenase, **characterized in that** the exchange takes place at position 576 of potato tuber lipoxygenase or at a corresponding position in a lipoxygenase of another plant species, **characterized in that** the exchange at position 576 leads to the presence of a Phe residue in the mutant.

2. The method according to claim 1, **characterized in that** the amino acid exchange is effected by directed mutagenesis.

3. Lipoxygenase obtainable by a method according to any one of claims 1 to 2.

4. Nucleic acid coding for a lipoxygenase according to claim 3.

5. Vector containing a nucleic acid according to claim 4.

6. Cell containing a nucleic acid according to claim 4 and/or a vector according to claim 5.

7. Plant or plant part comprising a host cell according to claim 6.

8. A method for producing 11-perhydroxy arachidonic acid or the reduced 11-hydroxy derivative, comprising the step of
- converting arachidonic acid with a lipoxygenase according to claim 5 and, optionally, reducing the perhydroxy compound obtained to hydroxy compound.

9. Use of a lipoxygenase according to claim 3 for producing 11-perhydroxy arachidonic acid and/or 11-hydroxy arachidonic acid.

## Revendications

1. Procédé pour l'augmentation de la spécificité d'une lipoxygénase végétale pour l'acide arachidonique en position 11, comprenant l'étape suivante :
Substitution d'au moins un acide aminé dans une lipoxygénase de type sauvage, **caractérisée en ce que** la substitution se fait en position 576 de la lipoxygénase d'un tubercule de pomme de terre ou en une position correspondante dans une lipoxygénase d'un autre type de plante, et **caractérisée en ce que** la substitution en position 576 entraîne la présence d'un résidu Phe dans le mutant.

2. Procédé selon la revendication 1, **caractérisé en ce que** la substitution d'acide aminé est effectuée par mutagenèse dirigée.

3. Lipoxygénase pouvant être obtenue par un procédé selon l'une des revendications 1 et 2.

4. Acide nucléique qui code pour une lipoxygénase selon la revendication 3.

5. Vecteur contenant un acide nucléique selon la revendication 4.

6. Cellule contenant un acide nucléique selon la revendication 4 et/ou un vecteur selon la revendication 5.

7. Plante ou partie d'une plante comprenant une cellule hôte selon la revendication 6.

8. Procédé pour la fabrication de 11-perhydroxy acide arachidonique et/ou du 11-hydroxydérivé réduit, comprenant l'étape suivante :
Conversion de l'acide aminé à l'aide d'une lipoxygénase selon la revendication 5 et le cas échéant réduction de la liaison perhydroxy obtenue en une liaison hydroxy.

9. Utilisation de la lipoxygénase selon la revendication 3 pour la fabrication de 11-perhydroxy acide arachidonique et/ou de 11-hydroxy acide arachidonique.


### SEQUENZPROTOKOLL

<110> Institut für Pflanzenbiochemie
<120> 11-Arachidonat-Lipoxygenase-Mutante
<130> P30743
<140>
   <141>
<160> 2
<170> PatentIn Ver. 2.1
<210> 1
   <211> 61
   <212> DNA
   <213> Künstliche Sequenz
<220>
   <223> Beschreibung der künstlichen Sequenz:primer
<400> 1
   gctggtgggg ttcttgagag tacattcttt ccttcgaaat ttgccatgga aatgtcagct 60
   g                                                                 61
<210> 2
   <211> 61
   <212> DNA
   <213> Künstliche Sequenz
<220>
   <223> Beschreibung der künstlichen Sequenz:primer
<400> 2
   cagcgtacat ttccatggca aatttcgaag gaaagaatgt actctcaaga accccaccag 60
   c                                                                 61